Europäisches Patentamt

European Patent Office

Office européen des brevets

(19)

(11) **EP 0 623 582 B1**

(12) **EUROPÄISCHE PATENTSCHRIFT**

(45) Veröffentlichungstag und Bekanntmachung des Hinweises auf die Patenterteilung:
**07.01.1998 Patentblatt 1998/02**

(51) Int. Cl.$^6$: **C07C 67/14**, C07C 69/63, C07C 67/08

(21) Anmeldenummer: **94106039.4**

(22) Anmeldetag: **19.04.1994**

(54) **Verfahren zur Herstellung von Carbonsäureestern aus Carbonsäurehalogeniden und Alkoholen**

Process for the preparation of esters of carboxylic acids from carboxylic acid halogenides and alcohols

Procédé de préparation d'esters d'acides carboxyliques à partir d'halogénures d'acides carboxyliques et d'alcools

(84) Benannte Vertragsstaaten:
**AT BE CH DE DK ES FR GB GR IE IT LI LU NL PT**

(30) Priorität: **27.04.1993 DE 4313791**
**03.03.1994 DE 4406997**

(43) Veröffentlichungstag der Anmeldung:
**09.11.1994 Patentblatt 1994/45**

(73) Patentinhaber:
**Solvay Fluor und Derivate GmbH**
**D-30173 Hannover (DE)**

(72) Erfinder:
• **Feist, Heinz Rudi**
**Greenwich, CT 06831 (US)**
• **Pohlmeyer, Wilhelm**
**D-30453 Hannover (DE)**
• **Frehse, Joachim**
**D-30625 Hannover (DE)**
• **Braun, Max**
**D-30900 Wedemark (DE)**
• **Eichholz, Kerstin**
**D-30855 Langenhagen (DE)**
• **Rudolph, Werner**
**D-30559 Hannover (DE)**

(74) Vertreter: **Lauer, Dieter, Dr.**
**Solvay Pharmaceuticals GmbH,**
**Hans-Böckler-Allee 20**
**30173 Hannover (DE)**

(56) Entgegenhaltungen:
**EP-A- 0 209 157**      **FR-A- 1 432 070**

**Beschreibung**

Die Erfindung bezieht sich auf ein Verfahren zur katalysierten Herstellung von Carbonsäureestern aus Carbonsäurehalogeniden und Alkoholen.

Viele Ester von Carbonsäuren werden in der Technik als solche verwendet. Essigester und andere Carbonsäureester dienen beispielsweise als Lösungs- oder Reinigungsmittel, andere Ester, z. B. von Bernsteinsäure, werden zur Aromatisierung eingesetzt. Der Trifluoressigsäureethylester ist beispielsweise ein Lösungsmittel für die Chlorierung von Paraffinen oder die Polymerisation von Olefinoxiden. Viele Carbonsäureester sind auch Zwischenprodukte in der chemischen Synthese. Der Trifluoressigsäuremethylester und Trifluoressigsäure-1.1.1-Trifluorethylester liefern nach Hydrierung Trifluorethanol (und ggf. Methanol). Trifluorethanol wird als Lösungsmittel und als Zwischenprodukt, beispielsweise bei der Herstellung des Lösungsmittels und Anästhetikums Isoflurane, verwendet. Ester der Trifluoressigsäure dienen auch zur Einführung bzw. zur Herstellung von biologisch wirksamen Verbindungen, die eine CF$_3$-Gruppe aufweisen. Beispielweise kann man durch N-Acylierung mit Trifluoressigsäuremethylester Peptide mit hormonaler Aktivität herstellen. Der Trifluorethylester liefert mit Kampfer-Derivaten Shiftreagenzien für die NMR-Analyse. Der Trifluoressigsäurephenylester liefert nach Fries'scher Verschiebung mit Aluminiumchlorid das entsprechende trifluoracetylierte Phenol, welches ein Synthesebaustein für Pharmazeutika ist. Viele weitere Anwendungszwecke von Estern sind dem Fachmann bekannt, beispielsweise die Umsetzung von Estern mit Aminen zu Amiden, die Synthesebausteine für Pharmazeutika, Photosensibilisatoren und Farbstoffe darstellen.

Ester der Chlordifluoressigsäure sind ebenfalls Synthesebausteine. Der Ethylester dient beispielsweise zum Aufbau von Flüssigkristallen, siehe DE-OS 4 023 106, bei der Herstellung von Arzneimitteln, siehe US-A 5 006 563, der Methylester ebenfalls für den Aufbau von Flüssigkristallen, als Ausgangsverbindung für die mikrobielle Herstellung chiraler sekundärer Alkohole, siehe T. Kitazume et al., J. Fluorine Chem. 56 (1992), Seiten 271 bis 284 oder für die Herstellung fluorierter Enolether in der Wittig-Synthese, siehe J. P. Beque et al., J. Org. Chem. 57 (1992), Seite 3807 ff. Die Ester der Chlordifluoressigsäure sind auch Vorstufen für Difluorcarben.

Die Herstellung von Carbonsäureestern erfolgt üblicherweise durch Umsetzung der entsprechenden Alkohole mit den Carbonsäuren unter saurer Katalyse. Dabei muß das entstehende Reaktionswasser zur Gleichgewichtsverschiebung entfernt werden; bei fluorierten Derivaten kann dies aufgrund der bevorzugten Bindung von Wasser (an den Carbonylfunktionen als Hydrate) zu Schwierigkeiten führen. Es ist auch bereits bekannt, daß man Carbonsäureester aus Carbonsäurechloriden und Alkoholen unter Basenkatalyse herstellen kann. Dabei ist jedoch eine hydrolytische Aufarbeitung notwendig, außerdem entstehen Abfallsalze, beispielsweise Pyridinhydrochlorid, welche entsorgt werden müssen. Die Umsetzung von Carbonsäurehalogeniden mit Alkoholen ohne Basenkatalyse verläuft mit geringer Reaktionsgeschwindigkeit.

Aufgabe der vorliegenden Erfindung ist es, ein technisch einfach durchführbares Verfahren anzugeben, mit welchem sich Carbonsäureester in hoher Ausbeute herstellen lassen, ohne daß Reaktionswasser abgetrennt werden muß oder eine hydrolytische Aufarbeitung notwendig ist. Diese Aufgabe wird durch das Verfahren der vorliegenden Erfindung gelöst. Das erfindungsgemäße Verfahren zur Herstellung von Carbonsäureestern aus Carbonsäurechloriden oder Carbonsäurebromiden und Alkoholen ist dadurch gekennzeichnet, daß man die Umsetzung wasserfrei in Anwesenheit eines Alkalimetall- oder "Onium"-Salzes der dem eingesetzten Carbonsäurechlorid oder Carbonsäurebromid entsprechenden Carbonsäure durchführt. Der gebildete Carbonsäureester kann dann z. B. destillativ abgetrennt werden.

Ein Zusatz von Säure, z. B. einer Carbonsäure, ist nicht notwendig und erfolgt vorzugsweise nicht.

Bevorzugt geht man von Carbonsäurechloriden aus. Als Katalysator verwendet man vorzugsweise ein "Onium"-Salz der Carbonsäure.

Das erfindungsgemäße Verfahren kann prinzipiell für die Herstellung beliebiger Ester von beliebigen Carbonsäuren mit beliebigen Alkoholen angewendet werden. Eine bevorzugte Ausführungsform sieht vor, daß man ein Carbonsäurechlorid der Formel R$^1$C(O)Cl (I) einsetzt, worin R$^1$ für Alkyl mit 1 bis 6 C-Atomen; durch mindestens 1 Halogenatom substituiertes Alkyl mit 1 bis 6 C-Atomen; Phenyl, Tolyl; durch mindestens 1 Halogenatom substituiertes Phenyl oder Tolyl steht.

Weiterhin ist es bevorzugt, daß man einen Alkohol der Formel R$^2$OH (II) einsetzt, worin R$^2$ für Alkyl oder Alkenyl mit 1 bis 8 C-Atomen; durch mindestens 1 Halogenatom substituiertes Alkyl oder Alkenyl mit 1 bis 8 C-Atomen; Phenyl, Tolyl; Benzyl; durch mindestens 1 Halogenatom und/oder mindestens eine Nitrogruppe substituiertes Phenyl, Tolyl oder Benzyl steht.

Ganz besonders bevorzugt ist es, daß R$^1$ für durch mindestens 1 Fluoratom substituiertes Alkyl mit 1 bis 4 C-Atomen und R$^2$ für Alkyl oder Alkenyl mit 1 bis 4 C-Atomen; durch mindestens 1 Halogenatom substituiertes Alkyl oder Alkenyl mit 1 bis 4 C-Atomen; Phenyl; durch mindestens 1 Halogenatom und/oder durch mindestens eine Nitrogruppe substituiertes Phenyl, steht. Insbesondere bedeutet R$^1$ Perfluormethyl, Perfluorethyl, Perfluorpropyl oder Chlordifluormethyl. Insbesondere bevorzugt steht R$^2$ für Alkyl oder Alkenyl mit 1 bis 3 C-Atomen; durch mindestens 1 Fluoratom substituiertes Alkyl oder Alkenyl mit 1 bis 3 C-Atomen; Phenyl; durch mindestens 1 Fluoratom und/oder mindestens

2

eine Nitrogruppe substituiertes Phenyl.

Als Alkalimetallsalz von Carbonsäuren sind beispielsweise Kalium- und Natriumsalze geeignet.

Der Begriff "Onium" steht für Kationen mit positiv geladenem Stickstoff, beispielsweise protonierte aromatische Stickstoffbasen wie Pyridinium oder protonierte Alkyl-, Dialkyl- oder Trialkylammonium-Kationen mit bis zu 20 C-Atomen, oder für durch Cycloalkyl substituierte Ammonium-Verbindungen oder cycloaliphatische Stickstoffbasen wie Piperidinium oder quartäre Ammoniumkationen.

Sehr gut geeignet als Carbonsäuresalz sind "Onium"-Salze, wobei "Onium" für ein Kation des Stickstoffs der Formel $R^I R^{II} R^{III} R^{IV} N^+$, worin $R^I$, $R^{II}$, $R^{III}$ und $R^{IV}$ unabhängig voneinander Wasserstoff, Alkyl mit 1 bis 20 C-Atomen, Aryl oder Aralkyl stehen, oder worin $R^I$ und $R^{II}$ oder worin $R^{III}$ und $R^{IV}$, oder worin $R^I$, $R^{II}$ und $R^{III}$ oder worin $R^I$, $R^{II}$, $R^{III}$ und $R^{IV}$, gegebenenfalls unter Einschluß des Stickstoff-Atoms, gesättigte oder ungesättigte Ringsysteme bilden. "Aryl" bedeutet hier insbesondere Phenyl oder durch 1 oder mehrere C1-C2-Alkylgruppen substituiertes Phenyl. Hervorragend geeignet sind Salze, in denen "Onium" für Ammonium, Pyridinium oder $R^{1'} R^{2'} R^{3'} R^{4'} N^+$ steht, worin $R^{1'}$, $R^{2'}$, $R^{3'}$ und $R^{4'}$ unabhängig voneinander Wasserstoff, Alkyl mit 1 bis 15 C-Atomen, Phenyl oder Benzyl stehen. Als Beispiel für solche Kationen seien genannt Pyridinium, Piperidinium, Anilinium, Benzyltriethylammonium und Triethylammonium.

Besonders gut geeignet ist das Verfahren zur Herstellung von Estern der durch ein oder mehrere Fluoratome substituierten Essigsäure. Beispielsweise kann man Trifluoressigsäurephenylester nach dem erfindungsgemäßen Verfahren herstellen. Besonders gut geeignet ist das erfindungsgemäße Verfahren zur Herstellung von Estern der Trifluoressigsäure oder Chlordifluoressigsäure mit 1.1.1-Trifluorethanol, Methanol, Ethanol, Isopropanol, 4-Nitro-Phenol, Pentafluorphenol und Allylalkohol.

Das Molverhältnis zwischen Carbonsäurehalogenid und Alkohol liegt vorteilhafterweise oberhalb von 0,9. Der Alkohol kann auch in höherem Überschuß eingesetzt werden und dient als Lösungsmittel, besonders wenn es sich um einen durch elektronenziehende Gruppen, beispielsweise Fluor-Atome, substituierten Alkohol handelt. Zweckmäßig liegt das Molverhältnis zwischen Alkohol und Carbonsäurehalogenid zwischen 0,9:1 und 1,1:1, bzw. falls der Alkohol als Lösungsmittel dient, bis hin zu 5:1.

Die Temperatur, bei der die Umsetzung durchgeführt wird, liegt bei Umgebungstemperatur (etwa 20 °C) bis hin zum Siedepunkt der Mischung, beispielsweise bis hin zu 100 °C. Man arbeitet bei Umgebungsdruck (etwa 1 bar abs.) oder gewünschtenfalls auch bei erhöhtem Druck, beispielsweise bei einem Druck von bis zu 5 bar abs.

Das Alkalimetall- oder "Onium"-Salz kann in katalytischen oder molaren Mengen anwesend sein. Zweckmäßig liegt das Mol-Verhältnis zwischen Säurehalogenid und dem Carbonsäuresalz im Bereich von 1:1 bis 20000:1.

Gemäß einer besonderen Ausführungsform der Erfindung erzeugt man das Säurechlorid bzw. das Säurebromid und das Alkalimetall- oder "Onium"-Salz der Carbonsäure in situ. Hierzu setzt man das entsprechende Alkalimetall- oder "Onium"-Halogenid, vorzugsweise das Chlorid oder Bromid, insbesondere das Chlorid, mit dem Anhydrid der einzusetzenden Carbonsäure um. Bei dieser Umsetzung bildet sich aus dem Anhydrid der Carbonsäure das entsprechende Säurehalogenid und das entsprechende Salz. Bei dieser Ausführungsform kann man als Alkalimetall- oder "Onium"-Halogenid verbrauchte Halogenid-Katalysatoren einsetzen, die auf diese Weise in Wertprodukte überführt werden können.

Vorzugsweise verwendet man "Onium"-Salz, insbesondere Pyridinium- oder Piperidinium-Salze.

Die Erfindung weist den Vorteil auf, daß man ohne hydrolytische A
ufarbeitung in technisch einfacher Weise Carbonsäure-ester erzeugen kann. Bei den meisten Estern fallen auch keine Abfallstoffe wie Pyridinhydrochlorid an.

Die Erfindung wird anhand der folgenden Beispiele weiter erläutert, ohne daß sie in ihrem Umfang eingeschränkt werden soll.

### Beispiel 1:

Herstellung von Trifluoressigsäure-trifluorethylester durch Umsetzung von Trifluoracetylchlorid mit 2,2,2-Trifluorethanol in Gegenwart von Pyridiniumtrifluoracetat

$$CF_3COCl + CF_3CH_2OH \xrightarrow{\text{PyTFA}} CF_3COOCH_2CF_3 + HCl$$

In einer Labor-Umlaufapparatur (1 l Vierhalskolben mit KPG-Rührer als Vorlage, Prominent-Pumpe, 30 cm Kolonne mit Raschig-Ringen gefüllt) wurden 30 g (0,16 mol) Pyridiniumtrifluoracetat in 355 g (3,55 mol) 2,2,2-Trifluorethanol aufgenommen und bei einer Innentemperatur von 54 °C im Umlauf geführt. Anschließend wurden in den Kolben über ein Tauchrohr 134 g (1,01 mol) Trifluoracetylchlorid innerhalb von 100 min zudosiert. Nach beendeter Trifluoracetylchloridzugabe ließ man 10 min nachreagieren. Anschließende fraktionierte Destillation des Reaktionsgemisches über eine Vigreuxkolonne (Übergangstemperatur: 54 bis 56 °C) ergab 177,8 g Trifluoressigsäure-trifluorethylester, dies entspricht einer Ausbeute von 89,8 % d. Theorie. Das im Vorlagekolben zurückgebliebene Trifluorethanol/Pyridiniumsalzgemisch kann erneut zur Veresterung mit gleicher Effektivität eingesetzt werden.

EP 0 623 582 B1

**Beispiel 2-7:**

Herstellung von Trifluoressigsäureestern durch Umsetzung von Trifluoracetylchlorid mit Methanol, Ethanol, Isopropanol, 4-Nitrophenol, Pentafluorphenol und Allylalkohol in Gegenwart von Pyridiniumtrifluoracetat

Analog der Vorschrift von Beispiel 1 wurden auch die entsprechenden Ester aus Trifluoracetylchlorid mit Methanol (94 %), Ethanol (96 %), Isopropanol (89 %), 4-Nitrophenol (85 %), Pentafluorphenol (92 %) und Allylalkohol (81 %) dargestellt.

**Beispiel 8:**

Herstellung von Trifluoressigsäure-trifluorethylester durch Umsetzung von Trifluoressigsäureanhydrid mit 2,2,2-Trifluorethanol in Gegenwart von Pyridiniumhydrochlorid

$$Py \cdot HCl + (CF_3CO)_2O + CF_3CO_2^- \cdot PyH + CF_3COCl \xrightarrow{CF_3CH_2OH} CF_3CO-OCH_2CF_3 + PyTFA + HCl$$

In einem 250 ml Dreihalskolben mit KPG-Rührer, Trockeneiskühler und Tropftrichter wurden 23,11 g (0,2 mol) Pyridiniumhydrochlorid und 20,0 g (0,2 mol) 2,2,2-Trifluorethanol vorgelegt. Anschließend tropfte man bei einer Reaktionsinnentemperatur von 52 bis 55 °C 42,01 g (0,2 mol) Trifluoressigsäureanhydrid innerhalb von 3 h zu. Die Ausbeute an Trifluoressigsäure-trifluorethylester betrug 98 % (GC). Die so erhaltene Reaktionslösung wurde entweder als Katalysatormischung für Versuche, wie in Beispiel 1 beschrieben, eingesetzt oder das 2,2,2-Trifluorethanol abdestilliert. Im Verlauf der Destillation reagierte im Destillationskolben die bei der Reaktion zunächst gebildete Trifluoressigsäure mit dem Pyridiniumhydrochlorid unter HCl-Bildung zu Pyridiniumtrifluoracetat ab.

**Beispiel 9:**

Herstellung von Chlordifluoressigsäure-methylester

$$CF_2ClC(O)Cl + CH_3OH \xrightarrow{CF_3CH_2OH} CF_2ClC(O)OCH_3 + HCl$$

Herstellung des Pyridiniumchlordifluoracetates: Unter $N_2$-Atmosphäre wurden in einem 250 ml Dreihalskolben mit Tropftrichter, KPG-Rührer und 40 cm Füllkörperkolonne zunächst 7,91 g (0,10 mol) Pyridin vorgelegt und 13,05 g (0,10 mol) Chlordifluoressigsäure (CDFA) unter schnellem Rühren (unter spontaner Erwärmung) langsam zugetropft.

Herstellung des Esters: Das entstandene Salz wurde in 32,0 g (1,0 mol) Methanol aufgenommen (das Salz löste sich sofort auf). Nach dem Erwärmen im Ölbad auf 50 °C tropfte man unter lebhafter HCl-Entwicklung 148,9 g (1,0 mol) Chlordifluoracetylchlorid zu. Eine gaschromatographische (GC)-Analyse der Reaktionsmischung zeigte nach Beendigung des Zutropfens eine quantitative Umsetzung des Säurechlorides zum Ester an. Die so erhaltene klare gelbe Lösung wurde anschließend über die 40 cm Füllkörperkolonne feindestilliert. Bei einer Sumpftemperatur von 80 - 85 °C und einer Übergangstemperatur von 78 - 79 °C erhielt man den Chlordifluoressigsäuremethylester in 99 %iger Reinheit.

**Beispiel 10:**

Herstellung von Chlordifluoressigsäure-ethylester

$$CF_2ClC(O)Cl + EtOH \xrightarrow{Py \cdot CDFA} CF_2ClC(O)OEt + HCl$$

Die Herstellung des Chlordifluoressigsäureethylesters erfolgte analog Beispiel 9. Man verwendete 10 Mol-% Pyridiniumchlordifluoracetat als Katalysator, 46,06 g (1,0 mol) Ethanol und 148,9 g (1,0 mol) Chlordifluoracetylchlorid. Nach der Zugabe von 0,5 mol des Säurechlorides war eine starke Eintrübung (Phasenbildung) der Reaktionslösung zu beobachten, die nach weiterer Säurechloridzugabe zum Reaktionsende hin wieder verschwand. Eine GC-Analyse nach Beendigung der HCl-Entwicklung zeigte wiederum einen quantitativen Umsatz des Säurechlorides zum Ester an. Die Reindestillation des Esters erfolgte über die 40 cm Füllkörperkolonne im Vakuum.

## Beispiel 11:

Herstellung von Chlordifluoressigsäure-propylester

$$CF_2ClC(O)Cl + PrOH \xrightarrow{Py \cdot CDFA} CF_2ClC(O)OPr + HCl$$

Die Herstellung des Chlordifluoressigsäurepropylesters erfolgte analog Beispiel 9. Man verwendete 10 Mol-% Pyridiniumchlordifluoracetat als Katalysator, 60,1 g (1,0 mol) n-Propanol und 148,9 g (1,0 mol) Chlordifluoracetylchlorid. Im Reaktionsverlauf war die Bildung zweier Phasen zu erkennen. Nach Beendigung der HCl-Entwicklung wurde die Rührung abgestellt und die Phasen mit GC-MS analysiert und charakterisiert. Die obere Phase enthielt neben Pyridiniumsalzen zu über 96 % den gewünschten Chlordifluoressigsäurepropylester, die untere Phase neben Spuren des Produktes die Katalysatorsalze als Hauptbestandteil. Der Umsatz des Säurechlorides zum Ester verlief quantitativ. Die Reindestillation des Esters erfolgte nach Phasentrennung über die 40 cm Füllkörperkolonne im Vakuum.

## Patentansprüche

1. Verfahren zur Herstellung von Carbonsäureestern aus Carbonsäurechloriden oder Carbonsäurebromiden und Alkoholen, wobei man die Umsetzung wasserfrei in Anwesenheit eines Alkalimetall- oder "Onium"-Salzes der dem eingesetzten Carbonsäurechlorid oder Carbonsäurebromid entsprechenden Carbonsäure durchführt.

2. Verfahren nach Anspruch 1, dadurch gekennzeichnet, daß man die Umsetzung ohne Zusatz von Carbonsäure durchführt.

3. Verfahren nach Anspruch 1 oder 2, dadurch gekennzeichnet, daß man ein Carbonsäurechlorid einsetzt.

4. Verfahren nach einem der vorhergehenden Ansprüche, dadurch gekennzeichnet, daß man ein Carbonsäurechlorid der Formel $R^1C(O)Cl$ (I) einsetzt, worin $R^1$ für Alkyl mit 1 bis 6 C-Atomen; durch mindestens 1 Halogenatom substituiertes Alkyl mit 1 bis 6 C-Atomen; Phenyl, Tolyl; durch mindestens 1 Halogenatom substituiertes Phenyl oder Tolyl steht.

5. Verfahren nach einem der vorhergehenden Ansprüche, dadurch gekennzeichnet, daß man einen Alkohol der Formel $R^2OH$ (II) einsetzt, worin $R^2$ für Alkyl oder Alkenyl mit 1 bis 8 C-Atomen; durch mindestens 1 Halogenatom substituiertes Alkyl oder Alkenyl mit 1 bis 8 C-Atomen; Phenyl, Tolyl; Benzyl; durch mindestens 1 Halogenatom und/oder mindestens eine Nitrogruppe substituiertes Phenyl, Tolyl oder Benzyl steht.

6. Verfahren nach Anspruch 4, dadurch gekennzeichnet, daß $R^1$ für durch mindestens 1 Fluoratom substituiertes Alkyl mit 1 bis 4 C-Atomen und $R^2$ für Alkyl oder Alkenyl mit 1 bis 4 C-Atomen; durch mindestens 1 Halogenatom substituiertes Alkyl oder Alkenyl mit 1 bis 4 C-Atomen; Phenyl; durch mindestens 1 Halogenatom und/oder durch mindestens eine Nitrogruppe substituiertes Phenyl steht.

7. Verfahren nach Anspruch 6, dadurch gekennzeichnet, daß $R^1$ für Perfluormethyl, Perfluorethyl, Perfluorpropyl oder Chlordifluormethyl steht.

8. Verfahren nach Anspruch 6, dadurch gekennzeichnet, daß $R^2$ für Alkyl oder Alkenyl mit 1 bis 3 C-Atomen; durch mindestens 1 Fluoratom substituiertes Alkyl oder Alkenyl mit 1 bis 3 C-Atomen; Phenyl; durch mindestens 1 Fluoratom und/oder mindestens eine Nitrogruppe substituiertes Phenyl steht.

9. Verfahren nach einem der vorhergehenden Ansprüche, dadurch gekennzeichnet, daß der Alkohol als Lösungsmittel eingesetzt wird.

10. Verfahren nach einem der vorhergehenden Ansprüche, dadurch gekennzeichnet, daß das Mol-Verhältnis zwischen Säurechlorid bzw. Säurebromid und Alkalimetall- oder "Onium"-Salz im Bereich von 1:1 bis 20000:1 liegt.

11. Verfahren nach einem der vorhergehenden Ansprüche, dadurch gekennzeichnet, daß man das Säurechlorid bzw. Säurebromid und das Alkalimetall- oder "Onium"-Salz in situ durch die Umsetzung des entsprechenden Alkalimetall- oder "Onium"-Chlorids oder -Bromids und dem Anhydrid der Carbonsäure erzeugt.

12. Verfahren nach einem der vorhergehenden Ansprüche, dadurch gekennzeichnet, daß man ein "Onium"-Salz, vorzugsweise ein Pyridinium- oder Piperidinium-Salz einsetzt.

## Claims

1. Process for preparing carboxylic esters from carboxylic acid chlorides or carboxylic acid bromides and alcohols, wherein the reaction is carried out in anhydrous medium in the presence of an alkali metal or "onium" salt of the carboxylic acid corresponding to the carboxylic acid chloride or carboxylic acid bromide used.

2. Process according to Claim 1, characterized in that the reaction is carried out without addition of carboxylic acid.

3. Process according to Claim 1 or 2, characterized in that a carboxylic acid chloride is used.

4. Process according to one of the preceding claims, characterized in that a carboxylic acid chloride of the formula $R^1C(O)Cl$ (I) is used, where $R^1$ is alkyl having from 1 to 6 carbon atoms; alkyl substituted by at least 1 halogen atom and having from 1 to 6 carbon atoms; phenyl, tolyl; phenyl or tolyl substituted by at least 1 halogen atom.

5. Process according to one of the preceding claims, characterized in that an alcohol of the formula $R^2OH$ (II) is used, where $R^2$ is alkyl or alkenyl having from 1 to 8 carbon atoms; alkyl or alkenyl substituted by at least 1 halogen atom and having from 1 to 8 carbon atoms; phenyl, tolyl; benzyl; phenyl, tolyl or benzyl substituted by at least 1 halogen atom and/or at least one nitro group.

6. Process according to Claim 4, characterized in that $R^1$ is alkyl substituted by at least 1 fluorine atom and having from 1 to 4 carbon atoms and $R^2$ is alkyl or alkenyl having from 1 to 4 carbon atoms; alkyl or alkenyl substituted by at least 1 halogen atom and having from 1 to 4 carbon atoms; phenyl; phenyl substituted by at least one halogen atom and/or by at least one nitro group.

7. Process according to Claim 6, characterized in that $R^1$ is perfluoromethyl, perfluoroethyl, perfluoropropyl or chlorodifluoromethyl.

8. Process according to Claim 6, characterized in that $R^2$ is alkyl or alkenyl having from 1 to 3 carbon atoms; alkyl or alkenyl substituted by at least 1 fluorine atom and having from 1 to 3 carbon atoms; phenyl; phenyl substituted by at least 1 fluorine atom and/or at least one nitro group.

9. Process according to one of the preceding claims, characterized in that the alcohol is used as solvent.

10. Process according to one of the preceding claims, characterized in that the molar ratio of acid chloride or acid bromide to alkali metal or "onium" salt lies in the range from 1:1 to 20,000:1.

11. Process according to one of the preceding claims, characterized in that the acid chloride or acid bromide and the alkali metal or "onium" salt is generated in situ by the reaction of the corresponding alkali metal or "onium" chloride or bromide and the anhydride of the carboxylic acid.

12. Process according to one of the preceding claims, characterized in that an "onium" salt, preferably a pyridinium or piperidinium salt, is used.

## Revendications

1. Procédé de préparation d'esters d'acides carboxyliques à partir de chlorures d'acides carboxyliques ou de bromures d'acides carboxyliques et d'alcools, selon lequel on effectue la réaction sans eau, en présence d'un sel de métal alcalin ou d'un sel d'"onium" de l'acide carboxylique correspondant au chlorure d'acide carboxylique ou au bromure d'acide carboxylique mis en oeuvre.

2. Procédé selon la revendication 1, caractérisé en ce que l'on effectue la réaction sans addition d'acide carboxylique.

3. Procédé selon la revendication 1 ou 2, caractérisé en ce qu'on utilise un chlorure d'acide carboxylique.

4. Procédé selon l'une des revendications précédentes, caractérisé en ce que l'on utilise un chlorure d'acide carboxylique de formule $R^1C(O)Cl$ (I), dans laquelle $R^1$ représente un groupe alkyle ayant 1 à 6 atomes de carbone; un groupe alkyle ayant 1 à 6 atomes et qui est substitué par au moins un atome d'halogène; un groupe phényle, tolyle; un groupe phényle ou tolyle substitué par au moins un atome d'halogène.

5. Procédé selon l'une des revendications précédentes, caractérisé en ce que l'on utilise un alcool de formule $R^2OH$ (II), dans laquelle $R^2$ représente un groupe alkyle ou alcényle ayant 1 à 8 atomes de carbone; un groupe alkyle ou alcényle ayant 1 à 8 atomes de carbone et qui est substitué par au moins un atome d'halogène; un groupe phényle ou tolyle; un groupe benzyle; un groupe phényle, tolyle ou benzyle substitué par au moins un atome d'halogène et/ou par au moins un groupe nitro.

6. Procédé selon la revendication 4, caractérisé en ce que $R^1$ représente un groupe alkyle ayant 1 à 4 atomes de carbone, substitué par au moins par un atome de fluor et $R^2$ représente un groupe alkyle ou alcényle ayant 1 à 4 atomes de carbone; un groupe alkyle ou alcényle ayant 1 à 4 atomes de carbone et qui est substitué par au moins un atome d'halogène; un groupe phényle; un groupe phényle substitué par au moins un atome d'halogène et/ou par au moins un groupe nitro.

7. Procédé selon la revendication 6, caractérisé en ce que $R^1$ représente un groupe perfluorométhyle, perfluoréthyle, perfluoropropyle ou chlorodifluorométhyle.

8. Procédé selon la revendication 6, caractérisé en ce que $R^2$ représente un groupe alkyle ou alcényle ayant 1 à 3 atomes de carbone; un groupe alkyle ou alcényle ayant 1 à 3 atomes de carbone et qui est substitué par au moins un atome de fluor; un groupe phényle, un groupe phényle substitué par au moins un atome de fluor et/ou par au moins un groupe nitro.

9. Procédé selon l'une des revendications précédentes, caractérisé en ce que l'alcool sert de solvant.

10. Procédé selon l'une des revendications précédentes, caractérisé en ce que le rapport molaire entre le chlorure d'acide ou le bromure d'acide et le sel de métal alcalin ou le sel d'"onium" se situe dans la gamme de 1:1 à 20 000:1.

11. Procédé selon l'une des revendications précédentes, caractérisé en ce que l'on produit le chlorure d'acide ou le bromure d'acide et le sel de métal alcalin ou le sel d'"onium" in situ par la réaction du chlorure ou du bromure de métal alcalin ou d'"onium" correspondant et de l'anhydride de l'acide carboxylique.

12. Procédé selon l'une des revendications précédentes, caractérisé en ce que l'on utilise un sel d'"onium", de préférence un sel de pyridinium ou de pipéridinium.